# EUROPEAN PATENT SPECIFICATION

(11) **EP 2 393 546 B1**
(45) Date of publication and mention of the grant of the patent: **03.04.2013**
(21) Application number: 10708361.0
(22) Date of filing: 04.02.2010
(51) Int. Cl.: A61M 31/00, A61M 3/02

(54) **CANNULA FOR DISPENSING FLUID PRODUCTS, PARTICULARLY FOR VAGINAL AND RECTAL APPLICATIONS**
KANÜLE ZUR ABGABE VON FLÜSSIGEN PRODUKTEN, INSBESONDERE FÜR VAGINALE UND REKTALE ANWENDUNGEN
CANULE DE DÉLIVRANCE DE PRODUITS FLUIDES, EN PARTICULIER POUR APPLICATIONS VAGINALES ET RECTALES

(30) Priority: 09.02.2009 IT MO20090031
(43) Date of publication of application: 14.12.2011
(73) Proprietor: LAMEPLAST S.p.A., Frazione Rovereto sul Secchia (MO) (IT)
(72) Inventor: FONTANA, Antonio, I-41012 Carpi (MO) (IT)
(74) Representative: Brunacci, Marco
(86) International application number: PCT/IB2010/000217
(87) International publication number: WO 2010/089653

(56) References cited:
- EP-A2- 1 518 574
- FR-A- 1 600 637
- US-A- 2 725 057
- US-A- 3 882 866
- US-A1- 2004 260 252

## Description

### Technical Field

The present invention relates to a cannula for dispensing fluid products, particularly for vaginal and rectal applications.

### Background art

Cannulas are known for dispensing medical fluid products which are used, in particular, for vaginal and rectal applications and which are generally sold in packs together with tubes or bottles containing the fluid products.

The known cannulas are composed of a cylinder suitable for containing the product and inside which is sliding a piston integral with the extremity of a push rod.

The cylinder has an open extremity that can be coupled with the dispenser mouth of the tube, to introduce into the cylinder itself the quantity of product to be applied, and through which the introduced product is dispensed.

The opposite extremity of the cylinder is closed by a bottom which has a hole, in which the push rod is inserted sliding, and which acts as a piston stop element to prevent its withdrawal.

The dispensing of the product introduced into the cannula occurs by moving the push rod in the sliding direction of the piston towards the open extremity of the cylinder.

These known cannulas have a number of drawbacks among which must be recalled that they are rather complex in terms of structure and build and that, being for hygienic reasons of the single-use type and requiring a push rod for each cylinder, they produce a considerable waste of materials.

To overcome such drawbacks, cannulas are known composed of one cylinder, which has the opposite extremities open and inside which a piston is fitted sliding, and of a push rod separated from the piston and removably coupled to it.

At the opposite extremities of the cylinder undercuts or stop shoulders are obtained suitable for stopping the sliding of the piston and preventing this from coming out as a result of the action of the push rod.

These latter cannulas can be sold in packs containing a single push rod, a plurality of empty single-use cylinders to be used for the different applications and one or more product tubes.

In this case one of the two extremities of the cylinders can be coupled with the dispenser mouth of the tube for the introduction of the product inside the cylinders themselves, while the opposite extremity acts as a passage for the push rod.

These latter known cannulas have made it possible to curb the consumption of materials; the same push rod, in fact, can be used with a plurality of cylinders. Nevertheless, these cannulas are not without drawbacks as well, tied above all to the need to use one or more tubes of product to load the cylinders before use. To overcome this drawback, an alternative type of so-called "pre-filled" cannula is known, meaning purchased with the cylinder already filled with the product to be applied and which has closing caps at the two opposite extremities which are removed at the time of use.

The pre-filled cannulas are not without drawbacks however, including the fact that they are of rather complex construction and that they have rather high production costs and times.

The cylinders, the pistons, the push rods and the closing caps in fact are normally made by moulding polymer materials, in several pieces (at least five) separated from one another and subsequently assembled; this involves the design and building of various moulds and laborious assembly operations which negatively affect the production costs and times of the cannulas, as well as producing considerable and costly material waste.

From the patent WO 2004/014476 on the other hand a particular type of pre-filled cannula is known in which the cylinder and the piston are made in a single body piece and, at an extremity of the cylinder, are joined together along a connection line with tearable weakened section.

The opposite extremity of the cylinder is closed by means of a tear-off film.

This particular type of pre-filled cannula also has a number of drawbacks however, such as, e.g., the fact that the fabrication of the piston in a single body piece with the cylinder is considerably complicated and hard to achieve in practice.

It should also be considered that big difficulties of a practical-manufacturing nature are also to be put down to the use of the tear-off film.

Another type of pre-filled cannula is on the other hand known from EP 1 518 574 and comprises a push rod that can be extended by means of a telescopic rod system between a retracted configuration and an elongated configuration.

The push rod is made in retracted configuration together with the cylinder at an extrernity of same.

The opposite extremity of the cylinder is instead closed by means of a cover cap made to fit over the cylinder permanently along its entire length until it joins at the base of the push rod.

The cannula described in EP 1 518 574 is also afflicted by various drawbacks, including the fact that the push rod involves the manufacture and the assembly of a plurality of telescopic elements, with consequent increase in times and costs of designing, production and assembly, as well as the waste of a considerable quantity of materials.

Another type of pre-filled cannula is on the other hand known from US 3,882,866 and comprises a disposable enema catheter having a barrel portion, open at one end, and a cannula affixed to the opposite end.

The barrel portion has a movable plug closing the barrel opening and the cannula has an enclosing cover.

The cover is removable from the cannula and acts as a plunger to move the plug against an enema solution contained in the barrel portion so that the solution is expelled through the cannula.

### Object of the Invention

The main aim of the present invention is to provide a cannula for dispensing fluid products, particularly for vaginal and rectal applications, which is structurally and constructively very simple, that allows considerably curbing the production times and costs and limiting the wastes of materials.

A further object of the present invention is to provide a cannula for dispensing fluid products, particularly for vaginal and rectal applications, which is of limited overall dimensions, is easy to handle and which can be immediately used by users.

Another object of the present invention is to provide a cannula for dispensing fluid products, particularly for vaginal and rectal applications, which allows to overcome the mentioned drawbacks of the known a in the ambit of a simple, rational, easy, effective to use and low cost solution.

The above objects are achieved by the resent cannula for dispensing fluid products, particularly for vaginal and rectal applications, according to claim 1.

### Brief Description of the Drawings

Other characteristics and advantages of the present invention will become more evident from the description of some preferred, but not sole, embodiments of a cannula for dispensing fluid products, particularly for vaginal and rectal applications, illustrated purely as an example but not limited to the annexed drawings in which:

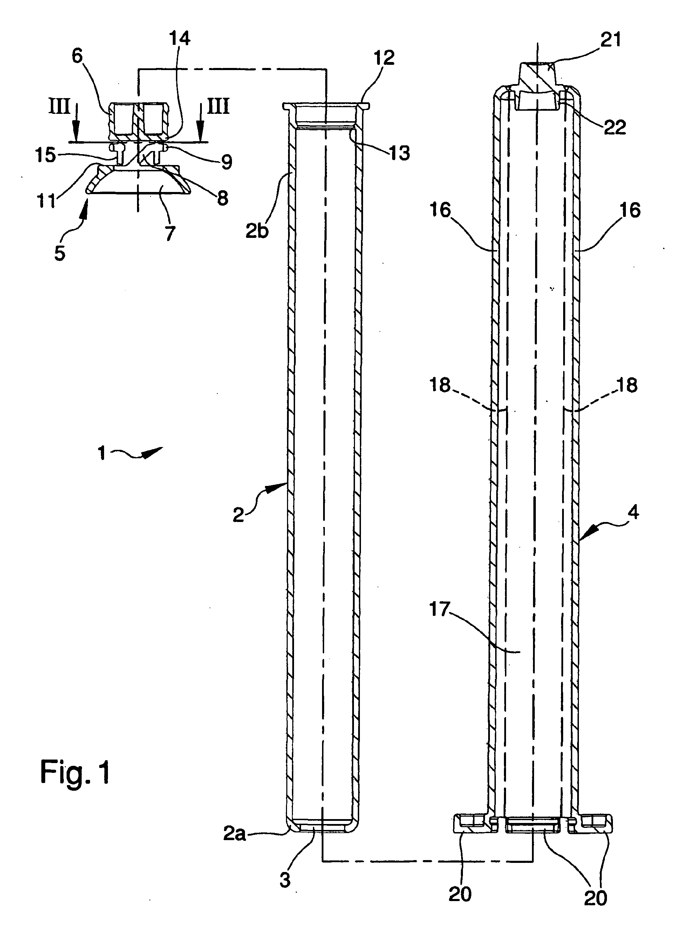

figure 1 is an exploded and section view of a first embodiment of the cannula according to the invention;
figure 2 is an axonometric view of the closing body of the cannula of figure 1;
figure 3 is a section view along the plane III - III of figure 1;
figure 4 is an axonometric view of the tubular body of the cannula of figure 1;
figure 5 is an axonometric view of cover cap of the cannula of figure 1;
figure 6 is a section view of the cannula of figure 1 in packaging confirguration;
figures from 7 to 9 show, in a sequence of axonometric views, the use method of the cannula of figure 1;
figure 10 is a section view of a second embodiment of the cannula according to the invention in packaging configuration.

### Embodiments of me Invention

With particular reference to the embodiment of the figures from 1 to 9, globally by 1 has been indicated a cannula for dispensing fluid products, particularly for vaginal and rectal applications.

In this respect, if is specified that in this treatise by the term fluid products are meant not only liquid products but also viscous products, e.g., in the state of paste and gel, and powdered products, in particular very fine powders distinguished by great flowability.

The cannula 1 comprises a tubular body 2 which is meant to contain the fluid product P.

The tubular body 2 is shaped like a straight cylinder and has a first extremity 2a at which is obtained at least a dispenser opening 3.

In the embodiment of the figures from 1 to 9 there is only one dispenser opening 3 which is obtained axially, meaning it extends crossways to the longitudinal direction of the tubular body 2.

Alternative embodiments of the invention are however possible in which several dispenser openings 3 are provided and/or in which these are obtained on the side surface of the first extremity 2a.

On the opposite side with respect to the first extremity 2a, the tubular body 2 has a second open extremity 2b.

The cannula 1 also comprises a cover cap 4 which is made separate from the tubular body 2 and which can be fitted on this to close the dispenser opening 3. To close the second extremity 2b, on the other hand, the cannula 1 comprises a closing body 5, this too made separate from the tubular body 2.

The closing body 5 comprises a first element 6 and a second element 7 made in a single body piece and joined along tearable connection means 8, 9, 10.

In detail, the second element 7 has a substantially round shape and can be torn off the first element 6 to act as a seal anti-tampering means, meaning to indisputably indicate that the cannula 1 has actually been opened by a user. The first element 6, on the other hand, has a substantially cylindrical shape and can be inserted snugly through the second extremity 2b to act as a piston sliding inside the tubular body 2 once the second element 7 has been torn off.

For this purpose, the cannula 1 has temporary retention means 11, 12, 13, 14 suitable for retaining the closing body 5 in a packaging configuration in which the first element 6 is inserted in the tubular body 2 near to the second extremity 2b.

The temporary retention means 11, 12, 13, 14, in particular, comprise a shoulder edge 11, which is obtained on the second element 7, and a corresponding locator edge 12, which is obtained around the second extremity 2b of the tubular body 2 and which can be engaged against the shoulder edge 11 to act as a stop and prevent fuller insertion of the closing body 5 in the tubular body 2

The temporary retention means 11, 12, 13, 14 also comprise a stop tooth 13, which is obtained in centripetal overhang on the inner surface of the tubular body 2, and a corresponding locator surface 14, which is obtained on the first element 6 and which can be engaged against the stop tooth 13 to prevent the withdrawal of the first element 6 from the tubular body 2.

The tearable connection means 8, 9, 10 comprise a removal arm 8, 9 stably joined to the second element 7 and connected to the first element 6 by interposition of a plurality of weakened-section connection points 10.

The removal arm 8, 9, e.g., is composed of a first portion 8, which extends substantially obliquely from the second element 7 and of a second portion 9, with substantially annular confirmation, which develops along a lying plane substantially at right angles to the longitudinal direction of the tubular body 2, and along which are distributed the connection points 10. Between the second portion 9 and the second element 7 stiffening bridges 15 are also provided which permit transmitting thrust forces from the second element 7 towards the first element 6.

The cover cap 4 is made in a single body piece with a push rod 25 insertable in the tubular body 2 to press the first element 6 sliding along the tubular body 2 and allow dispensing the fluid product P through the dispenser opening 3.

For this purpose, the cover cap 4 is split into a plurality of first longitudinal sections 16 and into at least a second longitudinal section 17 which can be torn of the first longitudinal sections 16.

The second longitudinal section 17, in particular, is joined to the first longitudinal sections 16 along weakened-section longitudinal connection lines 18 that extend substantially for the entire length of the cannula 1.

The first longitudinal sections 16, on the other hand, can be folded on themselves along longitudinal folding lines 19 that extend substantially along the entire length of the cannula 1.

Once the second longitudinal section 17 has been torn off, in practice, the first longitudinal sections 16 folded on themselves, define the push rod 25.

The transversal dimensions of the first longitudinal sections 16 folded on themselves are in fact considerably lower than the diameter of the cover cap 4 and such as to allow the insertion inside the tubular body 2 to push the first element 6.

In the embodiment shown in the figures from 1 to 9, therefore, the push rod 25 coincides with at least a part of the cover cap 4.

To make gripping by the user easier, the first longitudinal sections 16 and the second longitudinal section 17 have a gripping fin 20 obtained at the extremity of the cover cap 4 corresponding to the second extremity 2b of the tubular body 2.

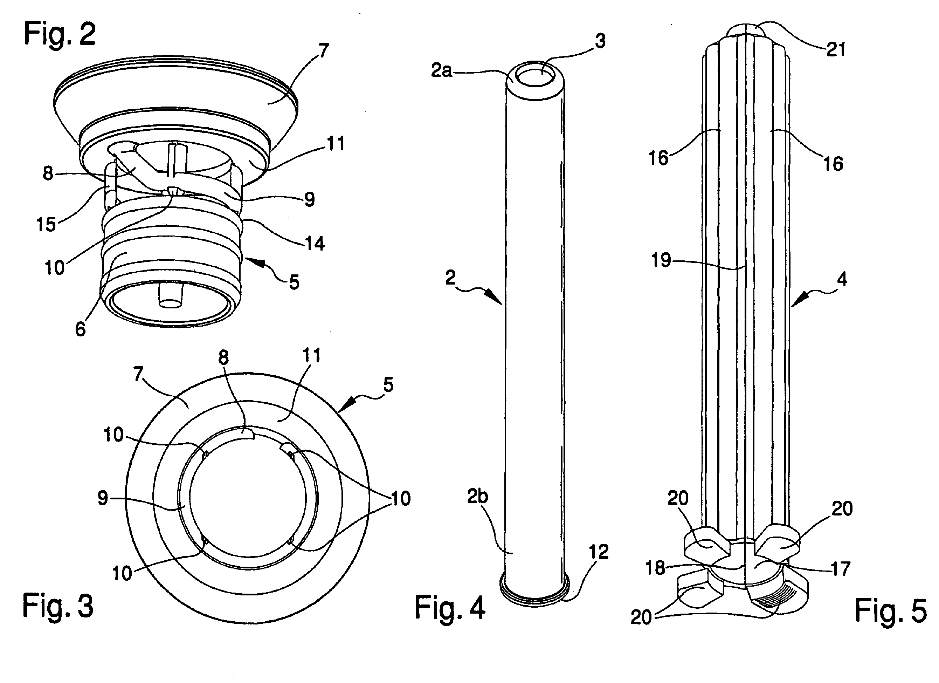

In particular, the gripping fins 20 of the first longitudinal sections 16 are distributed in an asymmetric way, as is clearly visible in the figure 5.

At the opposite extremity, the cover cap 4 has a bottom wall 21 which is substantially transversal to the longitudinal direction of the tubular body 2 and which can be fitted in front of the first extremity 2a.

At the bottom wall 21, the cover cap 4 comprises an inner shutter body 22 which can be inserted snugly in the dispenser opening 3.

Both the bottom wall 21 and the shutter body 22 are part of the second longitudinal section 17 and with this can be torn off the first longitudinal sections 16.

The operation of the cannula 1 shown in the figures from 1 to 9 is the following. The cannula 1 is made in just three separated pieces (tubular body 2, cover cap 4 and closing body 5) assembled together.

To make up the cannula 1 the cover cap 4 is first of all fitted on the tubular body 2, the tubular body 2 is pre-filled with the fluid product P and then the second extremity 2b is closed with the closing body 5.

The insertion of the closing body 5 in the tubular body 2 is done by pushing the second element 7 until the shoulder edge 11 is up against the locator edge 12 and the first element 6 is blocked on the stop tooth 13; in this phase, the thrust force is transmitted from the second element 7 to the first element 6 through the stiffening bridges 15.

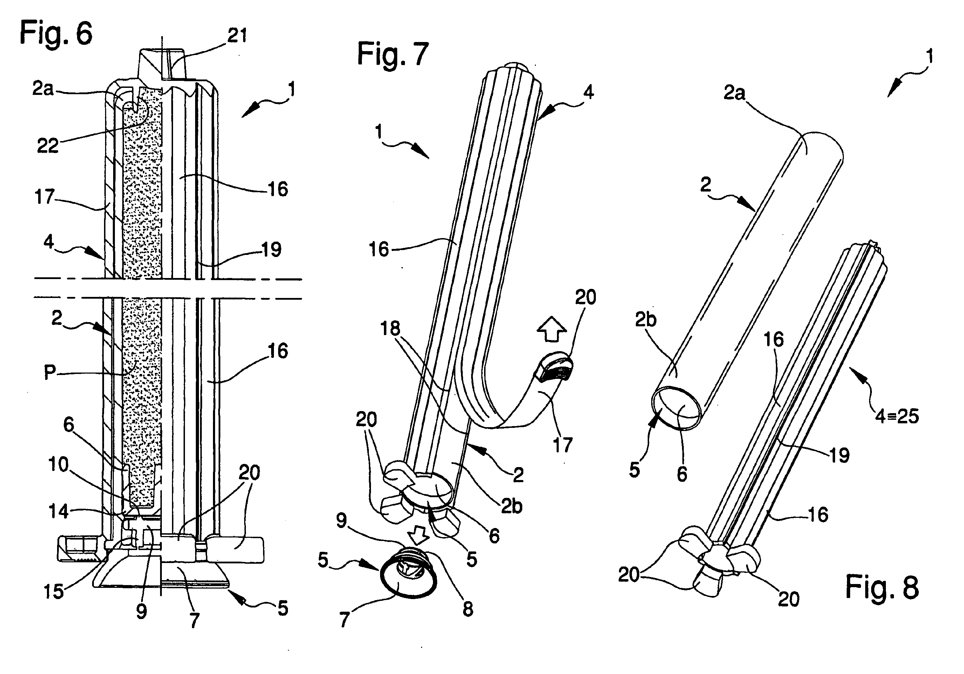

The cannula 1 is then distributed on the market in the packaging configuration shown in the figure 6, with the tubular body 2 pre-filled, covered by the cover cap 4 and sealed by the closing body 5.

At the time of use, the user removes the second element 7 and the cover cap 4 from the tubular body 2 (figure 7).

For this purpose, it is underlined that the removal of the second element 7 takes place simply by moving the second element 7 away from the second extremity 2b.

Such movement also drags outwards the removal arm 8, 9 which, by means of the first portion 8, is firmly joined to the second element 7.

The particular conformation of the removal arm 8, 9 thus allows tearing the connection points 10 in a sequential way; in other words, the annular shape of the second portion 9 causes the connection points 10 to be placed under tension and torn one after the other and not all at the same time.

This way, the force transmitted to the first element 6 is very limited and is easily discharged onto the stop tooth 13.

The removal of the cover cap 4, on the other hand, occurs by tearing the longitudinal connection lines 18.

For this purpose, all the user has to do is tear the second longitudinal section 17 by levering the corresponding gripping fin 20.

The second longitudinal section 17, in practice, also acts as a seal anti-tampering means as it indisputably shows whether the cannula 1 has been opened and, once torn, can be rejected as waste.

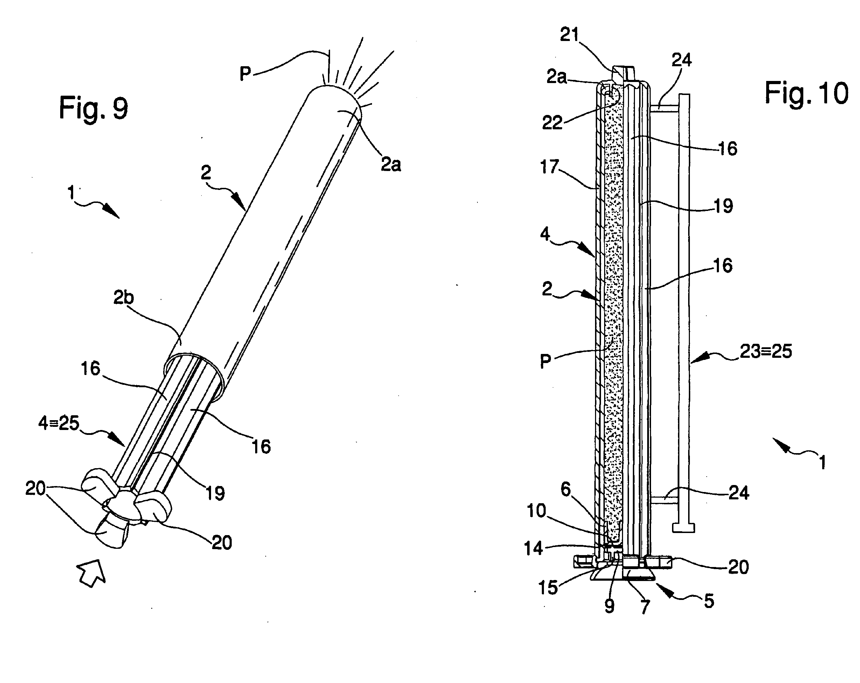

The first longitudinal sections 16, on the other hand, are folded on themselves along the longitudinal folding lines 19 (figure 8) and are used as push rod 25 to push the first element 6 sliding along the tubular body 2 (figure 9).

For this purpose, it is underlined that in this phase the particular asymmetric distribution of the gripping fins 20 easily permits folding the first longitudinal sections 16 superimposing at least in part the torn flaps, so as to facilitate the forming of the push rod 25 and its insertion in the tubular body 2.

In an alternative embodiment of the invention shown in the figure 10, the cannula I consists of a tubular body 2, a closing body 5 and a cover cap 4 substantially identical to those of the figures from 1 to 9, except for the fact that in this embodiment the push rod 25 does not coincide with the cover cap 4 but is composed of an elongated slat 23 joined to the cover cap 4 by interposition of a pair of tearable connection segments 24.

In the embodiment of figure 10 as well therefore, the cannula 1 is made in just three separated pieces in which, however, it is not the first longitudinal sections 16 of the cover cap 4 that act as a push rod 25, but the elongated slat 23.

The operation of this embodiment is substantially the same as that previously described and illustrated, with the difference that before use, the elongated slat 23 must be separated from the cover cap 4, tearing the connection segments 24, in order to use it as a push rod 25 to press the first element 6 along the tubular body 2.

It has in practice been found how the invention described achieves the intended objects.

In this respect, it is underlined that the present cannula for the dispensing of fluid products, particularly for vaginal and rectal applications, can be fabricated by assembly of just three components, permitting a considerable reduction in production times and costs, as well as of material wastes.

It is further pointed out that, thanks to a simple and compact structure, the cannula according to the present invention has low overall dimensions and is particularly simple and handy to use.

## Claims

1. Cannula (1) for dispensing fluid products, particularly for vaginal and rectal applications, comprising at least a tubular body (2) for containing at least a fluid product (P), having at least a first extremity (2a), at which at least a dispenser opening (3) is obtained, and a second open extremity (2b), opposed to the first extremity (2a), at least a closing body (5) which is associable with said tubular body (2) to close said second extremity (2b) and which comprises at least a first element (6) at least partially insertable through said second extremity (2b) and suitable for acting as a sliding piston inside said tubular body (2), and at least a cover cap (4) which can be fitted on said tubular body (2) to close said dispenser opening (3) and which is made in a single body piece with at least a push rod (25) insertable in said tubular body (2) to press said first element (6) sliding along said tubular body (2), **characterized by** the fact that said cover cap (4) is split into a plurality of first longitudinal sections (16) and into at least a second longitudinal section (17) removable by tearing off said first longitudinal sections (16).

2. Cannula (1) according to claim 1, **characterized by** the fact that at least one between said cover cap (4) and said closing body (5) is made separated from said tubular body (2).

3. Cannula (1) according to claim 1 or 2, **characterized by** the fact that said second longitudinal section (17) is joined to said first longitudinal sections (16) along Weakened-section longitudinal connection lines (18).

4. Cannula (1) according to claim 1 or 2 or 3, **characterized by** the fact that said first longitudinal sections (16) can be folded on themselves along longitudinal folding lines (19) to define said push rod (25) once said second longitudinal section (17) has been removed by tearing off.

5. Cannula (1) according to claim 1 or 2 or 3 or 4, **characterized by** the fact that at least one between said first longitudinal sections (16) and said second longitudinal section (17) has a gripping fin (20).

6. Cannula (1) according to claim 5, **characterized by** the fact that said gripping fin (20) is obtained at said second extremity (2b) of said tubular body (2).

7. Cannula (1) according to one or more of the preceding claims, **characterized by** the fact that said cover cap (4) comprises at least a bottom wall (21) substantially transversal and fittable in front of said first extremity (2a).

8. Cannula (1) according to one or more of the preceding claims, **characterized by** the fact that said cover cap (4) comprises at leat a shutter body (22) insertable in said dispenser opening (3).

9. Cannula (1) according to claim 7 or 8, **characterized by** the fact that at least one between said bottom wall (21) and said shutter body (22) is part of said second longitudinal section (17).

10. Cannula (1) according to one or more of the preceding claims, **characterized by** the fact that said push rod (25) comprises at least an elongated slat (23) joined to said cover cap (4) by interposition of at least a tearable connection segment (24).

11. Cannula (1) according to one or more of the preceding claims, **characterized by** the fact that said closing body (5) comprises at least a second element (7) made in a single body piece with said first element (6) and joined to it along tearable connection means (8, 9, 10), said second element (7) being removable by tearing off said first element (6) to act as a seal anti-tampering means and said first element (6) being suitable for acting as a sliding piston inside said tubular body (2) once said second element (7) has been torn off.

12. Cannula (1) according to claim 11, **characterized by** the fact that said tearable connection means (8, 9, 10) comprise at least a plurality of connection points (10) with weakened section.

13. Cannula (1) according to claim 12, **characterized by** the fact that said tearable connection means (8, 9, 10) comprise at least a removal arm (8, 9) stably joined to said second element (7) and joined to said first element (6) by interposition of said connection points (10).

14. Cannula (1) according to claim 13, **characterized by** the fact that said removal arm (8, 9) comprises at least one first portion (8) stably joined to said second element (7) and at least a second portion (9) which develops along a lying plane substantially at right angles to the longitudinal direction of said tubular body (2) and along which are distributed said connection points (10).

15. Cannula (1) according to claim 14, **characterized by** the fact that said second portion (9) has a substantially annular shape.

16. Cannula (1) according to one or more of the preceding claims, **characterized by** the fact that it comprises temporary retention means (11, 12, 13, 14) for retaining said closing body (5) in a packaging configuration in which said first element (6) is inserted in said tubular body (2) near to said second extremity (2b).

17. Cannula (1) according to claim 16, **characterized by** the fact that said temporary retention means (11, 12, 13, 14) comprise at least a stop tooth (13) obtained on at least one between said first element (6) and said tubular body (2) and at least a corresponding locator surface (14) obtained on the other between said first element (6) and said tubular body (2) and engageable against said stop tooth (13).

18. Cannula (1) according to claims 11 and 16, **characterized by** the fact that said temporary retention means (11, 12, 13, 14) comprise at least a shoulder edge (11) obtained on at least one between said second element (7) and said tubular body (2) and at least a corresponding locator edge (12) obtained on the other between said second element (7) and said tubular body (2) and engageable against said shoulder edge (11).

## Patentansprüche

1. Kanüle (1) zum Abgeben fluider Produkte, insbesondere für vaginale und rektale Anwendungen, die mindestens einen rohrförmigen Körper (2) zum Aufnehmen von mindestens einem fluiden Produkt (P), wobei der Körper (2) mindestens ein erstes Ende (2a), an dem mindestens eine Abgabeöffnung (3) ausgebildet ist, und ein zweites offenes Ende (2b), das dem ersten Ende (2a) gegenüber liegt, aufweist, mindestens einen Schließkörper (5), der mit dem rohrförmigen Körper (2) verbunden werden kann, um das zweite Ende (2b) zu verschließen, und mindestens ein erstes Element (6) aufweist, das mindestens teilweise durch das zweite Ende (2b) einsetzbar ist und geeignet ist, als ein gleitender Kolben innerhalb des rohrförmigen Körpers (2) zu wirken, und mindestens eine Abdeckkappe (4), die auf dem rohrförmigen Körper (2) angebracht werden kann, um die Abgabeöffnung (3) zu schließen, und einteilig mit mindestens einer Schubstange (25) ausgebildet ist, die in den rohrförmigen Körper (2) eingesetzt werden kann, um das erste Element (6) gleitend entlang des rohrförmigen Körpers (2) zu drücken, umfasst, **dadurch gekennzeichnet, dass** die Abdeckkappe (4) in mehrere erste Längsabschnitte (16) und in mindestens einen zweiten Längsabschnitt (17), der durch ein Abreißen der ersten Längsabschnitte (16) ablösbar ist, aufgeteilt ist.

2. Kanüle (1) nach Anspruch 1, **dadurch gekennzeichnet, dass** die Abdeckkappe (4) und/oder der Schließkörper (5) getrennt von dem rohrförmigen Körper (2) hergestellt sind.

3. Kanüle (1) nach Anspruch 1 oder 2, **dadurch gekennzeichnet, dass** der zweite Längsabschnitt (17) mit den ersten Längsabschnitten (16) entlang geschwächter Abschnitte längsseitiger Verbindungslinien (18) verbunden ist.

4. Kanüle (1) nach Anspruch 1 oder 2 oder 3, **dadurch gekennzeichnet, dass** die ersten Längsabschnitte (16) entlang längsseitiger Faltungslinien (19) auf sich selbst gefaltet werden können, um die Schubstange (25) zu definieren, sobald der zweite Längsabschnitt (17) durch Abreißen entfernt worden ist.

5. Kanüle (1) nach Anspruch 1 oder 2 oder 3 oder 4, **dadurch gekennzeichnet, dass** der erste Längsabschnitten (16) und/oder der zweite Längsabschnitt (17) einen Steg zum Greifen (20) aufweist.

6. Kanüle (1) nach Anspruch 5, **dadurch gekennzeichnet, dass** der Steg zum Greifen (20) an dem zweiten Ende (2b) des rohrförmigen Körpers (2) ausgebildet ist.

7. Kanüle (1) nach einem oder mehreren der vorhergehenden Ansprüche, **dadurch gekennzeichnet, dass** die Abdeckkappe (4) mindestens eine Bodenwand (21) umfasst, die im Wesentlichen transversal ist und vor dem ersten Ende (2a) anbringbar ist.

8. Kanüle (1) nach einem oder mehreren der vorhergehenden Ansprüche, **dadurch gekennzeichnet, dass** die Abdeckkappe (4) mindestens einen Verschlusskörper (22) umfasst, der in die Abgabeöffnung (3) eingesetzt werden kann.

9. Kanüle (1) nach Anspruch 7 oder 8, **dadurch gekennzeichnet, dass** die Bodenwand (21) und/oder der Verschlusskörper (22) ein Teil des zweiten Längsabschnitts (17) sind.

10. Kanüle (1) nach einem oder mehreren der vorhergehenden Ansprüche, **dadurch gekennzeichnet, dass** die Schubstange (25) mindestens einen länglichen Stab (23) umfasst, der mit der Abdeckkappe (4) über eine Zwischenanordnung von mindestens einem zerreißbaren Verbindungssegment (24) verbunden ist.

11. Kanüle (1) nach einem oder mehreren der vorhergehenden Ansprüche, **dadurch gekennzeichnet, dass** der Schließkörper (5) mindestens ein zweites Element (7) umfasst, das einteilig mit dem ersten Element (6) ausgebildet ist und mit diesem entlang der zerreißbaren Verbindungsmittel (8, 9, 10) verbunden ist, wobei das zweite Element (7) durch Abreißen des ersten Elements (6) ablösbar ist, um als ein Betrugsverhinderungs-Versiegelungsmittel zu wirken, und wobei das erste Element (6) geeignet ist, als ein gleitender Kolben innerhalb des rohrförmigen Körpers zu wirken (2), sobald das zweite Element (7) abgerissen ist.

12. Kanüle (1) nach Anspruch 11, **dadurch gekennzeichnet, dass** die zerreißbaren Verbindungsmittel (8, 9, 10) mindestens mehrere Verbindungspunkte (10) mit einem geschwächten Abschnitt umfassen.

13. Kanüle (1) nach Anspruch 12, **dadurch gekennzeichnet, dass** die zerreißbaren Verbindungsmittel (8, 9, 10) mindestens einen Entnahmearm (8, 9) umfassen, der mit dem zweiten Element (7) stabil verbunden und mit dem ersten Element (6) durch eine Zwischenanordnung der Verbindungspunkte (10) verbunden ist.

14. Kanüle (1) nach Anspruch 13, **dadurch gekennzeichnet, dass** der Entnahmearm (8, 9) mindestens einen ersten Abschnitt (8), der mit dem zweiten Element (7) stabil verbunden ist, und mindestens einen zweiten Abschnitt (9) umfasst, der entlang einer Lageebene im Wesentliche rechtwinklig zu der Längsrichtung des rohrförmigen Körpers (2) abgewickelt ist und längs dessen die Verbindungspunkte (10) verteilt sind.

15. Kanüle (1) nach Anspruch 14, **dadurch gekennzeichnet, dass** der zweite Abschnitt (9) eine im Wesentlichen ringförmige Form aufweist.

16. Kanüle (1) nach einem oder mehreren der vorhergehenden Ansprüche, **dadurch gekennzeichnet, dass** sie Mittel (11, 12, 13, 14) zum vorübergehenden Halten umfasst, um den Schließkörper (5) in einer Verpackungskonfiguration, in der das erste Element (6) in den rohrförmigen Körper (2) in der Nähe des zweiten Endes (2b) eingesetzt ist, zu halten.

17. Kanüle (1) nach Anspruch 16, **dadurch gekennzeichnet, dass** die Mittel (11, 12, 13, 14) zum vorübergehenden Halten mindestens einen Anschlagzahn (13), der an dem ersten Element (6) und/oder dem rohrförmigen Körper (2) ausgebildet ist, und mindestens eine entsprechende Lokalisierungsfläche (14) umfassen, die an dem anderen des ersten Elements (6) und des rohrförmigen Körpers (2) ausgebildet ist und gegen den Anschlagzahn (13) in Eingriff gebracht werden kann.

18. Kanüle (1) nach Anspruch 11 und 16, **dadurch gekennzeichnet, dass** die Mittel (11, 12, 13, 14) zum vorübergehenden Halten mindestens eine Schulterkante (11), die an dem zweiten Element (7) und/oder dem rohrförmigen Körper (2) ausgebildet ist, und mindestens eine entsprechende Lokalisierungskante (12) umfassen, die an dem anderen des zweiten Elements (7) und des rohrförmigen Körpers (2) ausgebildet ist und gegen die Schulterkante (11) in Eingriff gebracht werden kann.

## Revendications

1. Canule (1) pour délivrer des produits fluides destinés à des applications vaginales et rectales, comprenant au moins un corps tubulaire (2) destiné à contenir au moins un produit fluide (P), pourvu d'au moins une première extrémité (2a), sur laquelle est ménagée au moins une ouverture de distribution (3), et d'une deuxième extrémité ouverte (2b), opposée à la première extrémité (2a), au moins un corps de fermeture (5) qui est associable audit corps tubulaire (2) pour fermer ladite deuxième extrémité (2b) et qui comprend au moins un premier élément (6) insérable, au moins partiellement, dans ladite deuxième extrémité (2b) et apte à agir comme un piston coulissant à l'intérieur dudit corps tubulaire (2), et au moins un capuchon (4) qui peut être placé sur ledit corps tubulaire (2) pour fermer ladite ouverture de distribution (3) et qui est fait d'une seule pièce avec au moins une tige-poussoir (25) insérable dans ledit corps tubulaire (2) pour exercer une pression sur ledit premier élément (6) coulissant le long dudit corps tubulaire (2), ***caractérisée par le fait que*** ledit capuchon (4) est divisé en une pluralité de premières sections longitudinales (16) et en au moins une deuxième section longitudinale (17) pouvant être retirée par arrachage desdites premières sections longitudinales (16).

2. Canule (1) selon la revendication 1, ***caractérisée par** le fait qu*'au moins l'un d'entre ledit capuchon (4) et ledit corps de fermeture (5) est réalisé séparé dudit corps tubulaire (2).

3. Canule (1) selon la revendication 1 ou 2, ***caractérisée par le fait que*** ladite deuxième section longitudinale (17) est réunie auxdites premières sections longitudinales (16) le long de lignes de raccordement longitudinales (18) à section fragilisée.

4. Canule (1) selon la revendication 1, ou 2, ou 3, ***caractérisée par le fait que*** lesdites premières sections longitudinales (16) peuvent être repliées sur elles-mêmes le long de lignes de pliage longitudinales (19) pour définir ladite tige-poussoir (25) une fois que ladite deuxième section longitudinale (17) a été enlevée par arrachage.

5. Canule (1) selon la revendication 1, ou 2, ou 3, ou 4, ***caractérisée par** le fait qu*'au moins l'une d'entre lesdites premières sections longitudinales (16) et ladite deuxième section longitudinale (17) possède une ailette de préhension (20).

6. Canule (1) selon la revendication 5, ***caractérisée par le fait que*** ladite ailette de préhension (20) est réalisée au niveau de ladite deuxième extrémité (2b) dudit corps tubulaire (2).

7. Canule (1) selon l'une ou plusieurs des revendications précédentes, ***caractérisée par le fait que*** ledit capuchon (4) comprend au moins une paroi de fond (21) sensiblement transversale et pouvant être placée face à ladite première extrémité (2a).

8. Canule (1) selon l'une ou plusieurs des revendications précédentes, ***caractérisée par le fait que*** ledit capuchon (4) comprend au moins un corps obturateur (22) insérable dans ladite ouverture de distribution (3).

9. Canule (1) selon la revendication 7 ou 8, ***caractérisée par** le fait qu*'au moins l'un d'entre ladite paroi de fond (21) et ledit corps obturateur (22) fait partie de ladite deuxième section longitudinale (17).

10. Canule (1) selon l'une ou plusieurs des revendications précédentes, ***caractérisée par le fait que*** ladite tige-poussoir (25) comprend au moins une barrette allongée (23) réunie audit capuchon (4) par interposition d'au moins un segment de raccordement arrachable (24).

11. Canule (1) selon l'une ou plusieurs des revendications précédentes, ***caractérisée par le fait que*** ledit corps de fermeture (5) comprend au moins un deuxième élément (7) fait d'une seule pièce avec ledit premier élément (6) et réuni à lui le long de moyens de raccordement arrachables (8, 9, 10), ledit deuxième élément (7) pouvant être retiré par arrachage dudit premier élément (6) pour servir de moyens de fermeture inviolable et ledit premier élément (6) étant apte à agir comme un piston coulissant à l'intérieur dudit corps tubulaire (2) une fois que ledit deuxième élément (7) a été enlevé par arrachage.

12. Canule (1) selon la revendication 11, ***caractérisée par le fait que*** lesdits moyens de raccordement arrachables (8, 9, 10) comprennent au moins une pluralité de points de raccordement (10) à section fragilisée.

13. Canule (1) selon la revendication 12, ***caractérisée par le fait que*** lesdits moyens de raccordement arrachables (8, 9, 10) comprennent au moins un bras de séparation (8, 9) réuni de manière stable audit deuxième élément (7) et réuni audit premier élément (6) par interposition desdits points de raccordement (10).

14. Canule (1) selon la revendication 13, ***caractérisée par le fait que*** ledit bras de séparation (8, 9) comprend au moins une première portion (8) réunie de manière stable audit deuxième élément (7) et au moins une deuxième portion (9) qui se développe le long d'un plan sensiblement perpendiculaire à la direction longitudinale dudit corps tubulaire (2) et le long de laquelle sont distribués lesdits points de raccordement (10).

15. Canule (1) selon la revendication 14, ***caractérisée par le fait que*** ladite deuxième portion (9) est de forme sensiblement annuaire.

16. Canule (1) selon l'une ou plusieurs des revendications précédentes, ***caractérisée par le fait qu'**elle* comprend des moyens de retenue temporaire (11, 12, 13, 14) pour retenir ledit corps de fermeture (5) dans une configuration de conditionnement dans laquelle ledit premier élément (6) est inséré dans ledit corps tubulaire (2) près de ladite deuxième extrémité (2b).

17. Canule (1) selon la revendication 16, ***caractérisée par le fait que*** lesdits moyens de retenue temporaire (11, 12, 13, 14) comprennent au moins une dent d'arrêt (13) ménagée sur au moins l'un d'entre ledit premier élément (6) et ledit corps tubulaire (2), et au moins une surface de butée correspondante (14) ménagée sur l'autre d'entre ledit premier élément (6) et ledit corps tubulaire (2) et engageable contre ladite dent d'arrêt (13).

18. Canule (1) selon les revendications 11 et 16, ***caractérisée par le fait que*** lesdits moyens de retenue temporaire (11, 12, 13, 14) comprennent au moins un bord d'épaulement (11) ménagé sur au moins l'un d'entre ledit deuxième élément (7) et ledit corps tubulaire (2) et au moins un bord de positionnement correspondant (12) ménagé sur l'autre d'entre ledit deuxième élément (7) et ledit corps tubulaire (2) et engageable contre ledit bord d'épaulement (11).
